# EUROPEAN PATENT SPECIFICATION

(11) **EP 3 284 429 B1**
(45) Date of publication and mention of the grant of the patent: **06.11.2024**
(21) Application number: 17179081.9
(22) Date of filing: 28.12.2011
(51) Int. Cl.: A61B 17/92, A61B 17/16, A61F 2/46

(54) **ELECTRIC MOTOR DRIVEN TOOL FOR ORTHOPEDIC IMPACTING**
DURCH EINEN ELEKTROMOTOR ANGETRIEBENES WERKZEUG FÜR ORTHOPÄDISCHE EINGRIFFE
OUTIL ENTRAÎNÉ PAR MOTEUR ÉLECTRIQUE POUR IMPACT ORTHOPÉDIQUE

(30) Priority: 29.12.2010 US 201013980329
(43) Date of publication of application: 21.02.2018
(62) Divisional of application: 11854028.5
(73) Proprietor: DePuy Synthes Products, Inc., Raynham, MA 02767 (US)
(72) Inventor: PEDICINI, Christopher, Franklin, TN 37064 (US)
(74) Representative: Carpmaels & Ransford LLP

(56) References cited:
- WO-A2-2010/045158
- US-A- 4 298 074
- US-A- 5 057 112
- US-A- 5 352 230
- US-A- 5 485 887
- US-A- 974 267
- US-A1- 2005 116 673
- US-A1- 2008 215 056
- US-A1- 2009 065 226
- US-B1- 6 264 660

## Description

### FIELD

The present disclosure relates to electric tools for impacting in orthopedic applications, and, more particularly, to an electric motor driven tool for orthopedic impacting that is capable of providing controlled impacts to a broach, chisel, or other device for creating an opening in an area (in a bone structure, for example) to securely receive prosthesis within the area.

In the field of orthopedics, prosthetic devices such as artificial joints, are often implanted or seated in a patient's body by seating the prosthetic device in a cavity of a bone of the patient. The cavity must be created before the prosthesis is seated or implanted, and traditionally, a physician may remove worn, excess, or diseased bone structure from the area in which the cavity will be formed, and then drill and hollow out a cavity along the medullar canal of the bone. A prosthesis usually includes a stem or other protrusion that serves as the particular portion of the prosthesis that is inserted into the cavity.

To create such a cavity, a physician may use a broach, which broach conforms to the shape of the stem of the prosthesis. Solutions known in the art include providing a handle with the broach, which handle the physician may grasp while hammering the broach into the implant area. Unfortunately, this approach is clumsy and unpredictable as being subject to the skill of the particular physician. This approach almost will always inevitably result in inaccuracies in the location and configuration of the cavity. Further, this approach carries with it the risk that the physician will damage bone structure in unintended areas.

Another technique for creating the prosthetic cavity is to drive the broach pneumatically, that is, by compressed air. This approach is disadvantageous in that it prevents portability of an impacting tool, for instance, because of the presence of a tethering air line, air being exhausted from a tool into the sterile operating field and fatigue of the physician operating the tool. Further this approach, as exemplified in United States Patent 5,057,112 does not allow for precise control of the impact force or frequency and instead functions very much like a jackhammer when actuated. Again, this lack of any measure of precise control makes accurate broaching of the cavity more difficult.

Another disadvantage of tools known in the art is the accumulation of fluids, such as body fluids or moisture, on handgrips of such tools during prolonged periods of use. For example, difficulty of operation of a broach impacting device known in the art may increase during a surgical procedure as handgrips may become saturated with bodily fluids and thus the physician's hold on such a prior art device may become impaired.

Consequently, there exists a need for an impacting tool that overcomes the various disadvantages in the prior art.

United States Patent 5,485,887 describes a pneumatic impact tool for surgical use comprising a housing, and a tool holder held by the housing. The tool holder has a first section inside the housing and a second section outside the housing. The first section is provided at a free end with a head having first and second impact surfaces, and the second section is provided at a free end with a working tool attachment. A cylinder sits within the housing and a piston is provided within the cylinder and having an inner hollow space enclosing the head, the piston being driven by pneumatic pressure to oscillate within the cylinder. A manually operable switch is provided to slide the cylinder within the housing and relative to the head from a first position to a second position. In the first position, the oscillating piston impacts only on the first impact surface of the head and in the second position, the oscillating piston impacts only on the second impact surface of the head. In a third position of the switch, the supply of compressed air is ended, the piston is no longer driven and the impact tool does not generate strikes at all.

### SUMMARY

In view of the foregoing disadvantages of the prior art, an electric motor-driven orthopedic impacting tool configured to include all the advantages of the prior art, and to overcome the drawbacks inherent therein is provided. The tool may be used by orthopedic surgeons for orthopedic impacting in for example hips, knees, and shoulders. The tool is capable of holding a broach, chisel, or other device and gently tapping the broach, chisel or other device into the cavity with controlled percussive impacts, resulting in a better fit for the prosthesis or the implant. Further, the control afforded by such an electrically manipulated broach, chisel, or other device allows adjustment of the impact settings according to a particular bone type or other profile of a patient. The tool additionally enables proper seating or removal of the prosthesis or the implant into or out of an implant cavity. The tool is capable of applying cyclic impact forces on a broach, chisel, or other device, or an implant and of finely tuning impact force to a plurality of levels of impact force.

Accordingly, the present invention provides an orthopedic impacting tool for striking an object, the tool comprising a motor assembly comprising a motor and a linear motion converter, a piston operatively coupled to the motor assembly, the piston having a forward end and a rearward end, an air chamber, an impacting element, a control unit, a broach adapter capable of holding a broach, chisel, reamer, or other surgical implement, and an anvil element adapted to accept a portion of the broach adapter, the broach adapter being operatively coupled to the anvil element, a forward impact plate, and a rearward impact plate. The air chamber comprises a forward air chamber proximate to the forward end of the piston, and a rear air chamber proximate to the rearward end of the piston. The control unit is configured to direct the motor assembly to cause the linear motion converter to move the piston in cycles of forward and rearward reciprocating movement. Forward movement of the piston during the cycle and causes compression of air within the forward air chamber, and rearward movement of the piston during the cycle causes compression of air within the rear air chamber. The impacting element is caused to move to impact the forward impact plate upon application of compressed air from the forward air chamber, and the impacting element is caused to move to impact the rearward impact plate upon application of compressed air from the rear air chamber. The control unit is configured to allow for percussive impacting of the impacting element both on the forward impact plate and on the rearward impact plate in one cycle of the reciprocating movement of the piston. The control unit may include a force adjustment element, which element may control the impact force and avoid damage caused by uncontrolled impacts. The force may be regulated electronically or through the use of mechanical detents. The mechanical detent allows for an increased impact force without sacrificing control or precision of the broach machining operation. The motor assembly may comprise a voice coil motor.

The anvil element includes both a forward and rearward point of impact and may include a guide that constrains the striker to move in a substantially axial direction. In operation, the movement of the striker along the guide of the anvil element continues in either a forward or rearward direction until the striker hits the point of impact. As used in this context, "forward direction" connotes movement of the striker toward a broach or patient, and "rearward direction" connotes movement of the striker away from the broach or chisel or patient. If the impact point is at the front of the tool, i.e., in a forward direction, the impact causes the percussive force to be transmitted to a broach or chisel, pushing it further into the cavity. If the impact point is at the rear of the tool, the percussive force tends to pull the broach or chisel out of the cavity. The selectivity of either bidirectional or unidirectional impacting provides flexibility to a surgeon in either cutting or compressing material within the implant cavity, in that the choice of material removal or material compaction is often a critical decision in a surgical procedure. The impact point may be in the form of a plate that is disposed at an end or each end of the anvil element.

The tool may also be capable of regulating the frequency of the striker. By regulating the frequency of the striker, the tool may impart a greater total time-weighted percussive impact, while maintaining the same impact magnitude. This allows for the surgeon to control the cutting speed of the broach or chisel. For example, the surgeon may choose cutting at a faster rate (higher frequency impacting) during the bulk of the broach or chisel movement and then slow the cutting rate as the broach or chisel approaches a desired depth.

A user may firmly hold the tool by a handle portion and utilize light emitted by an LED to light up a work area and accurately position the broach, chisel, or other device on a desired location on the prosthesis or the implant. The reciprocating movement imparted on broach, chisel, or other device causes tapping of the implant and/or broach, chisel, or other device and thereby enables proper seating or removal of a prosthesis or implant into or out of an implant cavity, or controlled impacting of a broach, chisel, or other device to create or shape an implant cavity. The tool may also include a feedback system that warns the surgeon, when a bending or off-line orientation beyond a certain magnitude is detected at a broach, chisel, or other device/implant interface.

For a better understanding of the present disclosure, its operating advantages, and the specific objects attained by its uses, reference should be made to the accompanying drawing and descriptive matter.

### BRIEF DESCRIPTION OF THE DRAWINGS

The advantages and features of the present invention will become better understood with reference to the following detailed description and claims taken in conjunction with the accompanying drawings, wherein like elements are identified with like symbols, and in which:
Figure 1 shows a perspective view of an orthopedic impacting tool, in accordance with an exemplary embodiment of the present disclosure;
Figure 2 shows a perspective view of a compression piston compressing air against a striker of an orthopedic impacting tool, in accordance with an exemplary embodiment of the present disclosure;
Figure 3 shows a striker releasing and striking an anvil element, in accordance with an exemplary embodiment of the present disclosure;
Figure 4 shows a compression piston of an orthopedic impacting tool returning from a forward position and compressing air on the forward side of a striker, in accordance with an exemplary embodiment of the present disclosure;
Figure 5 shows a striker of an orthopedic impacting tool moving rearward, in accordance with an exemplary embodiment of the present disclosure; and
Figure 6 shows a striker of an orthopedic impacting tool impacting a rear anvil impact plate, in accordance with an exemplary embodiment of the present disclosure.

Like references numerals refer to like parts throughout the description of several views of the drawings.

### DETAILED DESCRIPTION OF THE DISCLOSURE

The best mode for carrying out the present disclosure is presented in terms of its preferred embodiment, herein depicted in the accompanying figures. The preferred embodiments described herein detail for illustrative purposes are subject to many variations.

The terms "a" and "an" herein do not denote a limitation of quantity, but rather denote the presence of at least one of the referenced items.

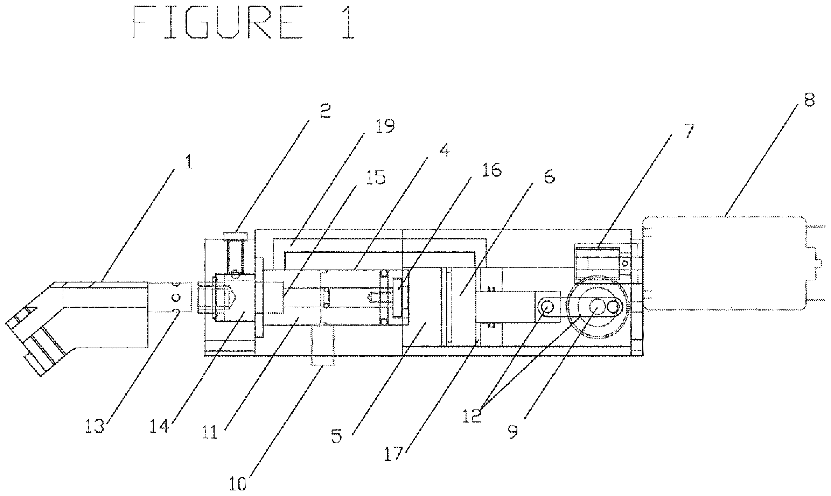

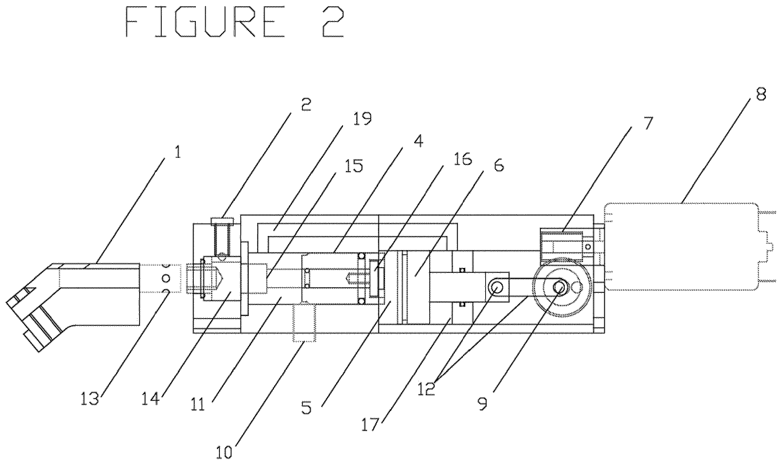

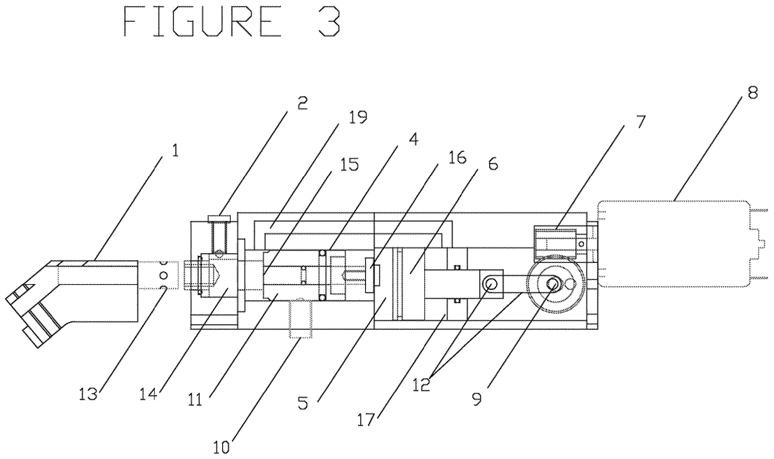

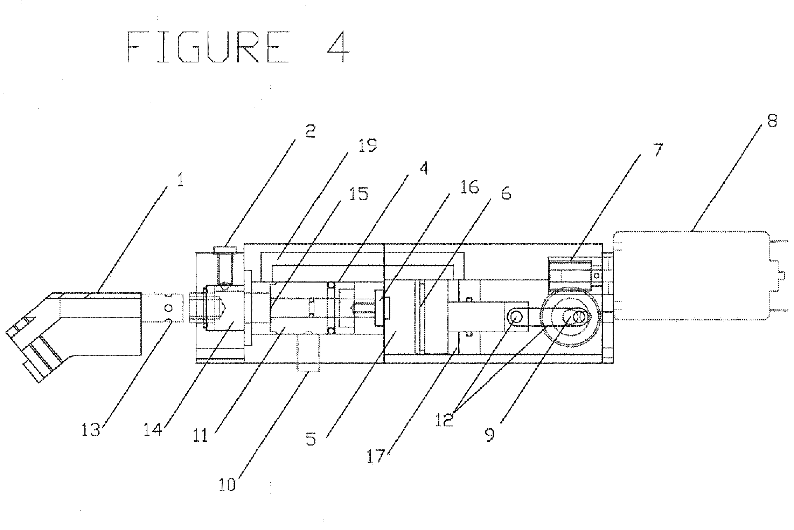

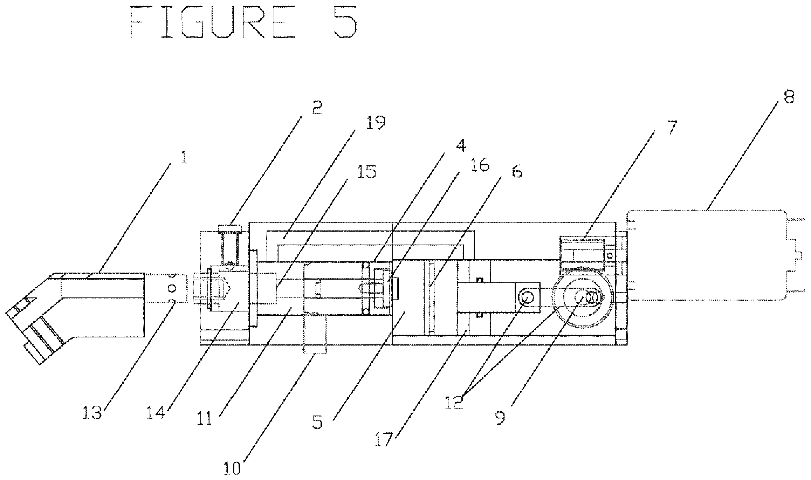

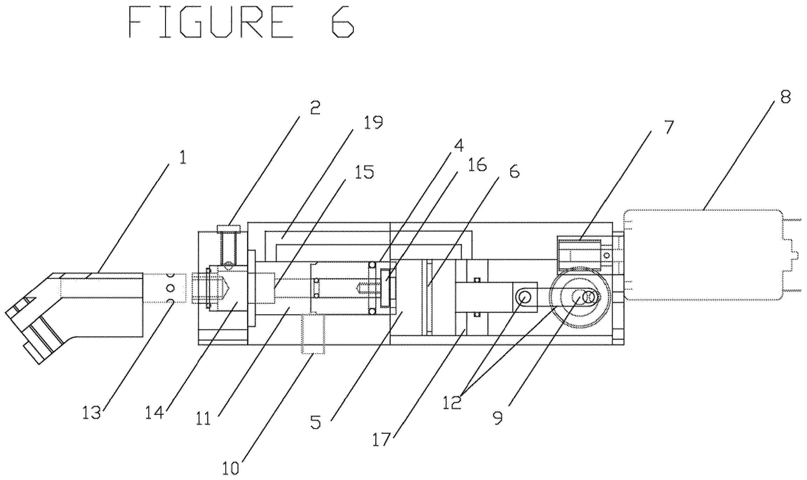

Referring now to FIGS. 1-6, the present disclosure provides an electric motor-driven orthopedic impacting tool with controlled percussive impacts. The tool includes the capability to perform single or multiple impacts as well as impacting of variable speeds, forces and frequencies. The impact force can be tuned to one of a variety of levels by setting the impact force either electronically or through the use of detents. The detent facilitates the generation of a sufficient strike force to make a meaningful impact in the broach, chisel, or other device, or surgical area.

The tool includes a housing. The housing securely covers and holds a plurality of components of the tool. In an embodiment, the housing contains a motor, at least one reducing gear, a linear motion converter, a compression chamber, an impacting element (alternately referred to as a striker), a force or impact adjustment control means (hereinafter referred to as 'control means'), and an anvil element with a forward impact plate and a rearward impact plate (which impact plates may be part of the anvil, for example).

The tool further may include a handle portion with at least one hand grip for comfortable and secure holding of the tool while in use, a broach adapter, a battery, and a positional sensor, a directional sensor, and/or a torsion sensor. The tool may further comprise an lighting element such as an LED to provide light in the work area in which a user employs the tool. The broach adapter can be coupled to an anvil, of the anvil element for example, through a quick connect mechanism at the end of the tool that is directed at a patient when the tool is in use.

In an embodiment, and referring now to FIG. 1, the linear motion converter comprises a slider crank mechanism 12, which slider crank is operatively coupled to the motor 8 and reducing gears 7 and 9. The tool further comprises an air chamber 5, 17 that accepts a compression piston 6 with a first end and a second end, which compression piston 6 is actuated by the linear motion converter 12. As the compression piston will have a smaller longitudinal dimension than the air chamber that contains the piston, it will be apparent that an air mass will be present at either end of the compression piston while the compression piston is within the air chamber. Hereinafter, the air mass disposed between the head of compression piston and the striker will be referred to as the "forward air chamber portion" or "forward air chamber," 5 and the air mass disposed between the end of the compression piston that is proximate to the linear motion converter and the motor of the tool will be referred to as the "rear air chamber portion" or "rear air chamber" 17.

In an embodiment of the present disclosure the motor of the tool causes the linear motion converter to move the compression piston until sufficient pressure is built within the forward air chamber 5 that is disposed between the forward end of the compression piston and the rearward end of the striker 4 to either overcome a detent 10 that otherwise retains the striker in a rearward position and/or the inertia and frictional force that holds the striker 4 in a position. Once this sufficient pressure is reached, the force of the air pressure accelerates the striker 4, which striker slides axially down a cavity internal to the tool housing and strikes the forward anvil impact plate 15. The resultant force is communicated through the anvil 14 that is proximate to the impact plate 15 and, optionally, through the broach adapter 1 (which adapter will be described in more detail below) to which a broach, chisel, or other device for seating or removing an implant, or prosthesis may be attached.

As the compression piston 6 continues through its stroke it moves towards the rear direction, compressing the air mass in the rear air chamber 17. This air mass may be communicated through an air passageway 19 to the front side of the striker 4, creating a returning force on the striker 4, which returning force causes the striker 4 to move in a rear direction, i.e., a direction away from the point of impact of the striker the forward anvil impact plate 15. The striker continues to move until it impacts the rear anvil impact plate 16. Striking the rear impact plate creates a rear directed force on the anvil 14. In the event that a broach adapter 1 is attached to the anvil 14, the force is communicated through the broach adapter 1 to which the broach, chisel, or other device for seating or removing an implant, or prosthesis is attached. Thus in one complete cycle, a forward and rear directed impacting force can be applied on the broach, chisel, or other device, or implant/prosthesis.

In an embodiment, the compression piston preferably has a cavity on the head thereof, which cavity creates pressure during the return stroke of the piston, which pressure causes the front end of the striker to move away from the forward anvil impact plate impact the rearward point of impact of the anvil element. It will be apparent that the striker impacting the rear anvil impact plate will communicate a negative force to the front of the anvil (and broach, chisel, or other device), which negative force will move the broach, chisel, or other device away from the location of impact in a surgical area.

The slider crank embodiment of the tool facilitates controlled continuous impacting of the striker and anvil. For such continuous impacting, after causing compression by the compression piston, the slider crank returns to the bottom of its stroke, which return releases pressure on the striker and, in the above-described embodiment wherein the piston comprises a cavity on the head thereof, may pressurize the front of the striker, causing the striker to return to its initial rest position.

For a single stroke, the linear motion converter (such as the slider crank described herein) will stop at or near the rear position, thus releasing the forward pressure on the striker and allowing the striker to return it to its starting position in readiness for another stroke. In this operational mode, a user may cause the tool to impact selectively (as opposed to repeatedly), thus allowing further control of the impacts and the creation or shaping of the surgical area, for example.

A positional sensor, coupled operatively to the control unit may be provided to assist in regulating a preferred positional cyclic operation of the linear motion converter. For example, the control unit may cause the slider crank to come to rest at or near the fully back position in readiness to generate pressure for the next impact upon receiving a signal from the positional sensor that the slider crank has reached the bottom dead center position. In another embodiment, the control unit may be directly coupled to the linear motion converter for initiating and ceasing operation of the linear motion converter.

The control unit is further capable of operating the force control means for selectively tuning the amount of impact force per cycle. By controlling the impact force the tool can avoid damage caused by uncontrolled impacts or impacts of excessive force. For example, a user may reduce the impact setting in the case of an elderly patent with osteoporosis, or may increase the impact setting for more resilient or intact athletic bone structures.

One such force control preferably comprises a selectable release setting on a mechanical detent that holds the striker. It will be apparent that the striker will impact the anvil with greater force in the case where the pressure needed to dislodge the striker from the detent is increased. In another embodiment, the detent may comprise a solenoid, which solenoid may activate upon a predetermined buildup of pressure, after which the striker 4 is released, allowing it to impact the anvil.

The control unit may also control the force of impacting by modulating the speed of advancing (forward directional travel) and/or the speed of retraction (rearward directional travel) of the compression piston. It will be apparent that the modulation of speed of the compression piston will affect the buildup of pressure for forward and rearward directional travel of the striker. For example, where the speed of the forward direction of the piston is relatively high, and the speed of the rearward direction of the piston is relatively low, the velocity of the striker in the forward direction will be much higher, causing the imparted percussive impact of the striker to be greater in the forward direction of the piston and striker. This modulation of the speed of the piston in the forward and rearward direction allows a user to create a greater impacting force, when so desired (e.g., to create a surgical area) or a greater rearward force, to facilitate removing a broach, chisel, or other device from the surgical area, for example. In the instance where the forward and rear velocities are the same, the tool allows for bidirectional movement of the broach, chisel, or other device during operation, which creates a very efficient technique for machining the cavity.

The motor of the tool may be configured to assist particularly with such multidirectional impacting. In an embodiment, the motor may operate under pulse-width modulation for rearward striking and may operate under full or continuous speed for forward striking of the striker. In such operation, the broach, chisel, or other device attached to the tool may undergo near forward only motion, which operation will facilitate the creation of an implant seat. Alternatively, the motor may operate under pulse-width modulation for forward striking and may operate under full or continuous speed for rearward impacting, which operation can create an extraction motion useful for dislodging a broach, chisel, or other device that has become stuck or removing an implant.

In a further embodiment, the tool comprises a positional sensor, such as an anvil positional sensor that may be operatively coupled to the control unit of the striker of the tool. This positional sensor is capable of determining whether the operator is pushing or pulling on the tool. For instance, the sensor may determine such pushing or pulling based upon the position of the broach-holding adapter or anvil. This determination can have the effect that when a user is exerting force on the tool in a particular direction the impacting of the striker is accordingly adjusted. For example, if the sensor determines that the user is pushing on the tool or is pushing the tool against an object, that sensor can cause the striker to impact in a forward direction. If the sensor determines that the user is pulling on the tool, that sensor may cause the striker to impact in a rearward direction or may cause a pulling force to be exerted on the striker by way of the cycling of the slider crank.

The tool may further comprise a lighting element, and, in an embodiment, the lighting element may comprise an LED arrangement, which lighting element may be capable of illuminating a user's work area. In an embodiment, the LED may be disposed on the housing of the tool and may be oriented toward a patient's body or surgical cavity.

The tool may further comprise a plate or other flat surface at the end of the tool that is distal to the surgical area, which plate may allow a user to apply selective manual pressure on a broach, chisel or other device, or a surgical implant as dictated by surgical or physical conditions. For instance, if a broach is firmly lodged within a cavity such that the operation of the tool would not remove the broach, the user may manually tap on the plate to dislodge the broach.

The tool may further comprise a torsion sensor, which torsion sensor may be capable of determining a lateral or deviating force or movement of the tool, such that if the tool is sensed to deviate from a pre-determined magnitude at the broach/implant interface, a signal may emit to notify the user of such deviation. In this manner and otherwise, the tool facilitates consistent axial broaching and implant seating.

In a further embodiment, the broach adapter may comprise a parallel 4-bar arrangement. In this embodiment, the adapter may receive a broach for anterior or posterior joint replacement. The parallel 4-bar mechanism of the adapter may facilitate receiving and orienting the broach in a variety of positions, such as in a centered position, or in an offset left or right position. The adapter will maintain the broach in an orientation that is parallel or co-linear to the body of the tool and the striker. The broach adapter may also comprise clamps, a vice, or any other fastener that may securely hold the broach, chisel, or other device, during operation of the tool.

The tool may further comprise handgrips disposed on the housing of the tool, which handgrips may include a rubberized or other tacky coating removably disposed thereon. Such coating facilitates comfortable operation of the tool and improves the user's hold on the tool for increased control thereof and reduced fatigue during operation of the tool.

In use, a user such as a surgeon firmly holds the tool by the handle grip or grips and utilizes light emitted by the LED to illuminate a work area and accurately position a broach, chisel or other device that has been attached to the tool on a desired location on the prosthesis or implant. The reciprocating movement imparted by the tool upon the broach, chisel or other device causes tapping of the implant and thereby enables proper seating or removal of the prosthesis or the implant into or out of an implant cavity. The warning system may alert the user in the event that a bending moment above a certain magnitude is detected at a broach (or chisel or other device)/implant interface.

The tool disclosed herein provides various advantages over the prior art. It facilitates controlled impacting at a surgical site, which minimizes unnecessary damage to a patient's body and which allows precise shaping of a implant or prosthesis seat. The tool also allows the user to modulate the direction and force of impacts, which improves the user's ability to manipulate the tool. The force control adjustment of the impact settings allows a user to set the force of impact according to a particular bone type or other profile of a patient. The tool thereby enables proper seating or removal of the prosthesis or the implant into or out of an implant cavity.

The foregoing descriptions of specific embodiments of the present disclosure have been presented for purposes of illustration and description. They are not intended to be exhaustive or to limit the present disclosure to the precise forms disclosed, and obviously many modifications and variations are possible in light of the above teaching. The exemplary embodiment was chosen and described in order to best explain the principles of the present disclosure and its practical application, to thereby enable others skilled in the art to best utilize the disclosure and various embodiments with various modifications as are suited to the particular use contemplated.

## Claims

1. An orthopedic impacting tool for striking an object, the tool comprising:
a motor assembly (8, 12) comprising a motor (8) and a linear motion converter (12);
a piston (6) operatively coupled to the motor assembly (8, 12), the piston (6) having a forward end and a rearward end;
an air chamber;
an impacting element (4);
a control unit;
a broach adapter (1) capable of holding a broach, chisel, reamer, or other surgical implement; and
an anvil element (14) adapted to accept a portion of the broach adapter (1), the broach adapter (1) being operatively coupled to the anvil element (14);
a forward impact plate (15); and
a rearward impact plate (16);
wherein the air chamber comprises a forward air chamber (5) proximate to the forward end of the piston (6), and a rear air chamber (17) proximate to the rearward end of the piston (6);
wherein the control unit is configured to direct the motor assembly (8, 12) to cause the linear motion converter (12) to move the piston in cycles of forward and rearward reciprocating movement;
wherein forward movement of the piston (6) during the cycle will cause compression of air within the forward air chamber (5);
and wherein rearward movement of the piston (6) during the cycle will cause compression of air within the rear air chamber (17);
wherein the impacting element (4) will be is caused to move to impact the forward impact plate (15) upon application of compressed air from the forward air chamber (5); and wherein the impacting element (4) will be caused to move to impact the rearward impact plate (16) upon application of compressed air from the rear air chamber (17);
wherein the control unit is configured to allow for percussive impacting of the impacting element (4) both on the forward impact plate (15) and on the rearward impact plate (16) in one cycle of the reciprocating movement of the piston.

2. The orthopedic impacting tool of claim 1, wherein:
the orthopedic impacting the tool further comprises an air passageway (19) adjacent to the air chamber, the air passageway (19) being capable of communicating air from the air chamber to the impacting element (4);
wherein the compression of the air within the rear air chamber (17) will be communicated through the passageway (19) and onto the impacting element (4), such that the compressed air communicated through the air passageway (19) will cause the impacting element (4) to impact the rearward impact plate (16);
wherein impacts on the forward and rearward impact plates (15, 16) will impart a force upon the broach adapter (1) in first and second directions, the second direction being substantially collinear and opposite to the first direction.

3. The orthopedic impacting tool of any preceding claim, wherein the motor assembly (8, 12) comprises a slider crank mechanism.

4. The orthopedic impacting tool of any preceding claim, wherein the motor assembly (8, 12) comprises a voice coil motor.

5. The orthopedic impacting tool of any preceding claim, wherein:
the orthopedic impacting tool comprises a positional sensor operatively coupled to one of the control unit of the orthopedic impacting tool and the motor assembly (8, 12) of the orthopedic impacting tool, wherein the sensor is configured to determine whether the tool is being pushed toward an object or pulled away from an object, the sensor is configured to cause the control unit or motor assembly (8,12) of the orthopedic impacting tool to direct the impacting element (4) to impart a greater force upon the forward impact plate (15) and a lesser force on the rearward impact plate (16) upon sensing that the tool is being pushed toward an object, and the sensor is configured to cause the control unit or motor assembly (8, 12) of the orthopedic impacting tool to direct the impacting element (4) to impart a greater force upon the forward impact plate (15) and a lesser force on the rearward impact plate (16) upon sensing that the tool is being pushed away from an object.

6. The orthopedic impacting tool of any preceding claim, wherein the tool comprises a torsion sensor configured to:
determine a lateral movement of the orthopedic impacting tool; and
signal such lateral movement.

7. The orthopedic impacting tool of any preceding claim, wherein the orthopedic impacting tool further comprises a lighting element disposed on an exterior surface of the orthopedic impacting tool, the lighting element operatively coupled to the control unit.

8. The orthopedic impacting tool of any preceding claim, wherein the velocity of the impacting element (4) can be controlled by the speed at which the air is compressed or decompressed.

9. The orthopedic impacting tool of any preceding claim, wherein the broach adapter (1) has an adjustment configured to offset an attached broach, chisel, reamer, or other surgical implement from the central axis of the orthopedic impacting tool while maintaining a parallel orientation of the broach, chisel, reamer, or other surgical implement with respect to the central axis of the orthopedic impacting tool.

10. The orthopedic impacting tool of claim 1, further comprising a detent (10) for retaining the impacting element (4) in a position.

11. The orthopedic impacting tool of claim 10, wherein:
the control unit is configured to direct the motor assembly (8, 12) to cause movement of the piston (6) and compression of air within the air chamber, so that when the pressure of the compressed air exceeds a force of the detent (10), the impacting element (4) will be is caused to move from a first position to a second position, thereby striking the forward (15) or rearward (16) impact plate.

12. The orthopedic impacting tool of claim 10 or 11, wherein a retaining force of said detent (10) can be controlled by a solenoid that is operatively coupled to the control unit.

13. The orthopedic impacting tool any of claims 10 to 12, further comprising a force adjustment control operatively coupled to the control unit, wherein the force adjustment control is operable to at least one of changing the speed at which said piston (6) moves within said air chamber, and changing said retention force of the detent (10).

14. The orthopedic impacting tool of any preceding claim, wherein the broach adapter (1) is proximate to the forward impact plate (15) of the anvil element (14) and distal to the rearward impact plate (16) of the anvil element (14).

## Patentansprüche

1. Orthopädisches Schlagwerkzeug für das Schlagen auf ein Objekt, wobei das Werkzeug umfasst:
eine Motoranordnung (8, 12), die einen Motor (8) und einen Linearbewegungswandler (12) umfasst;
einen Kolben (6), der mit der Motoranordnung (8, 12) wirkgekoppelt ist, wobei der Kolben (6) ein vorderes Ende und ein hinteres Ende aufweist;
eine Luftkammer;
ein Schlagelement (4);
eine Steuereinheit;
einen Räumnadeladapter (1), der eine Räumnadel, einen Meißel, eine Reibahle oder ein anderes chirurgisches Instrument halten kann; und
ein Ambosselement (14), das zum Aufnehmen eines Abschnitts des Räumnadeladapters (1) angepasst ist, wobei der Räumnadeladapter (1) mit dem Ambosselement (14) wirkgekoppelt ist;
eine vordere Schlagplatte (15); und
eine hintere Schlagplatte (16);
wobei die Luftkammer eine vordere Luftkammer (5) in der Nähe des vorderen Endes des Kolbens (6) und eine hintere Luftkammer (17) in der Nähe des hinteren Endes des Kolbens (6) umfasst;
wobei die Steuereinheit konfiguriert ist, um die Motoranordnung (8, 12) anzuweisen, den Linearbewegungswandler (12) dazu zu veranlassen, den Kolben in Zyklen von vorwärts- und rückwärtsgerichteter Hin- und Herbewegung zu bewegen;
wobei die Vorwärtsbewegung des Kolbens (6) während des Zyklus eine Kompression der Luft in der vorderen Luftkammer (5) bewirkt;
und wobei die Rückwärtsbewegung des Kolbens (6) während des Zyklus eine Kompression der Luft in der hinteren Luftkammer (17) bewirkt;
wobei bewirkt wird, dass sich das Schlagelement (4) bewegt, um auf die vordere Schlagplatte (15) bei Anwendung von Druckluft aus der vorderen Luftkammer (5) zu schlagen;
und wobei bewirkt wird, dass sich das Schlagelement (4) bewegt, um auf die hintere Schlagplatte (16) bei Anwendung von Druckluft aus der hinteren Luftkammer (17) zu schlagen;
wobei die Steuereinheit konfiguriert ist, um ein perkussives Schlagen des Schlagelements (4) sowohl auf die vordere Schlagplatte (15) als auch auf die hintere Schlagplatte (16) in einem Zyklus der Hin- und Herbewegung des Kolbens zu ermöglichen.

2. Orthopädisches Schlagwerkzeug nach Anspruch 1, wobei:
das orthopädische Schlagwerkzeug ferner einen an die Luftkammer angrenzenden Luftdurchlass (19) umfasst, wobei der Luftdurchlass (19) in der Lage ist, Luft von der Luftkammer an das Schlagelement (4) weiterzuleiten;
wobei die Kompression der Luft innerhalb der hinteren Luftkammer (17) durch den Durchlass (19) und auf das Schlagelement (4) weitergeleitet wird, so dass die durch den Luftdurchlass (19) weitergeleitete Druckluft bewirkt, dass das Schlagelement (4) auf die hintere Schlagplatte (16) schlägt;
wobei Schläge auf die vordere und hintere Schlagplatte (15, 16) eine Kraft auf den Räumnadeladapter (1) in einer ersten und einer zweiten Richtung ausüben, wobei die zweite Richtung im Wesentlichen kollinear und entgegengesetzt zur ersten Richtung ist.

3. Orthopädisches Schlagwerkzeug nach einem der vorstehenden Ansprüche, wobei die Motoranordnung (8, 12) einen Schubkurbelmechanismus umfasst.

4. Orthopädisches Schlagwerkzeug nach einem der vorstehenden Ansprüche, wobei die Motoranordnung (8, 12) einen Schwingspulenmotor umfasst.

5. Orthopädisches Schlagwerkzeug nach einem der vorstehenden Ansprüche, wobei:
das orthopädische Schlagwerkzeug einen Positionssensor umfasst, der entweder mit der Steuereinheit des orthopädischen Schlagwerkzeugs oder der Motoranordnung (8, 12) des orthopädischen Schlagwerkzeugs wirkgekoppelt ist, wobei der Sensor konfiguriert ist, um zu bestimmen, ob das Werkzeug zu einem Objekt hin gedrückt oder von einem Objekt weg gezogen wird, wobei der Sensor konfiguriert ist, um zu bewirken, dass die Steuereinheit oder Motoranordnung (8, 12) des orthopädischen Schlagwerkzeugs das Schlagelement (4) anweist, eine größere Kraft auf die vordere Schlagplatte (15) und eine geringere Kraft auf die hintere Schlagplatte (16) auszuüben, wenn erfasst wird, dass das Werkzeug in Richtung eines Objekts gedrückt wird, und der Sensor konfiguriert ist, um zu bewirken, dass die Steuereinheit oder Motoranordnung (8, 12) des orthopädischen Schlagwerkzeugs das Schlagelement (4) anweist, eine größere Kraft auf die vordere Schlagplatte (15) und eine geringere Kraft auf die hintere Schlagplatte (16) auszuüben, wenn erfasst wird, dass das Werkzeug von einem Objekt weg gedrückt wird.

6. Orthopädisches Schlagwerkzeug nach einem der vorstehenden Ansprüche, wobei das Werkzeug einen Torsionssensor umfasst, der konfiguriert ist zum:
Bestimmen einer seitlichen Bewegung des orthopädischen Schlagwerkzeugs; und
Signalisieren dieser seitlichen Bewegung.

7. Orthopädisches Schlagwerkzeug nach einem der vorstehenden Ansprüche, wobei das orthopädische Schlagwerkzeug ferner ein Beleuchtungselement umfasst, das auf einer Außenoberfläche des orthopädischen Schlagwerkzeugs angeordnet ist, wobei das Beleuchtungselement mit der Steuereinheit wirkgekoppelt ist.

8. Orthopädisches Schlagwerkzeug nach einem der vorstehenden Ansprüche, wobei die Geschwindigkeit des Schlagelements (4) durch die Geschwindigkeit gesteuert werden kann, mit der die Luft komprimiert oder dekomprimiert wird.

9. Orthopädisches Schlagwerkzeug nach einem der vorstehenden Ansprüche, wobei der Räumnadeladapter (1) eine Einstellung aufweist, die konfiguriert ist, um eine angebrachte Räumnadel, einen Meißel, eine Reibahle oder ein anderes chirurgisches Instrument von der Mittelachse des orthopädischen Schlagwerkzeugs zu versetzen, während eine parallele Ausrichtung der Räumnadel, des Meißels, der Reibahle oder des anderen chirurgischen Instruments in Bezug auf die Mittelachse des orthopädischen Schlagwerkzeugs beibehalten wird.

10. Orthopädisches Schlagwerkzeug nach Anspruch 1, ferner umfassend eine Feststellvorrichtung (10) zum Zurückhalten des Schlagelements (4) in einer Position.

11. Orthopädisches Schlagwerkzeug nach Anspruch 10, wobei:
die Steuereinheit konfiguriert ist, um die Motoranordnung (8, 12) anzuweisen, eine Bewegung des Kolbens (6) und eine Kompression der Luft innerhalb der Luftkammer zu bewirken, so dass, wenn der Druck der Druckluft eine Kraft der Feststellvorrichtung (10) übersteigt, das Schlagelement (4) dazu veranlasst wird, sich von einer ersten Position zu einer zweiten Position zu bewegen, wodurch es auf die vordere (15) oder hintere (16) Schlagplatte schlägt.

12. Orthopädisches Schlagwerkzeug nach Anspruch 10 oder 11, wobei eine Rückhaltekraft der Feststellvorrichtung (10) von einer Magnetspule gesteuert werden kann, die mit der Steuereinheit wirkgekoppelt ist.

13. Orthopädisches Schlagwerkzeug nach einem der Ansprüche 10 bis 12, ferner umfassend eine Kraftanpassungssteuerung, die mit der Steuereinheit wirkgekoppelt ist, wobei die Kraftanpassungssteuerung zum mindestens einem von Ändern der Geschwindigkeit, mit der sich der Kolben (6) innerhalb der Luftkammer bewegt, und Ändern der Rückhaltealtekraft der Feststellvorrichtung (10) betreibbar ist.

14. Orthopädisches Schlagwerkzeug nach einem der vorstehenden Ansprüche, wobei sich der Räumnadeladapter (1) in der Nähe der vorderen Schlagplatte (15) des Ambosselements (14) und distal der hinteren Schlagplatte (16) des Ambosselements (14) befindet.

## Revendications

1. Outil d'impact orthopédique pour frapper un objet, l'outil comprenant :
un ensemble moteur (8, 12) comprenant un moteur (8) et un convertisseur de mouvement linéaire (12) ;
un piston (6) accouplé de manière fonctionnelle à l'ensemble moteur (8, 12), le piston (6) ayant une extrémité avant et une extrémité arrière ;
une chambre à air ;
un élément d'impact (4) ;
une unité de commande ;
un adaptateur de broche (1) pouvant contenir une broche, un ciseau, un alésoir ou un autre instrument chirurgical ; et
un élément d'enclume (14) adapté à recevoir une partie de l'adaptateur de broche (1), l'adaptateur de broche (1) étant accouplé de manière fonctionnelle à l'élément d'enclume (14) ;
une plaque d'impact avant (15) ; et
une plaque d'impact arrière (16) ;
dans lequel la chambre à air comprend une chambre à air avant (5) proche de l'extrémité avant du piston (6), et une chambre à air arrière (17) proche de l'extrémité arrière du piston (6) ;
dans lequel l'unité de commande est configurée pour ordonner à l'ensemble moteur (8, 12) d'amener le convertisseur de mouvement linéaire (12) à déplacer le piston dans des cycles de mouvement alternatif vers l'avant et vers l'arrière ;
dans lequel un mouvement vers l'avant du piston (6) pendant le cycle provoquera une compression de l'air dans la chambre à air avant (5) ;
et dans lequel le mouvement vers l'arrière du piston (6) pendant le cycle provoquera une compression de l'air dans la chambre à air arrière (17) ;
dans lequel l'élément d'impact (4) sera amené à se déplacer pour réaliser un impact sur la plaque d'impact avant (15) lors de l'application d'air comprimé à partir de la chambre à air avant (5) ;
et dans lequel l'élément d'impact (4) sera amené à se déplacer pour réaliser un impact sur la plaque d'impact arrière (16) lors de l'application d'air comprimé à partir de la chambre à air arrière (17) ;
dans lequel l'unité de commande est configurée pour permettre un impact percutant de l'élément d'impact (4) à la fois sur la plaque d'impact avant (15) et sur la plaque d'impact arrière (16) au cours d'un cycle du mouvement alternatif du piston.

2. Outil d'impact orthopédique selon la revendication 1, dans lequel :
l'outil d'impact orthopédique comprend en outre un passage d'air (19) adjacent à la chambre à air, le passage d'air (19) pouvant communiquer l'air de la chambre à air à l'élément d'impact (4) ;
dans lequel la compression de l'air dans la chambre à air arrière (17) sera communiquée par le passage (19) et sur l'élément d'impact (4), de telle sorte que l'air comprimé communiqué par le biais du passage d'air (19) amènera l'élément d'impact (4) à réaliser un impact sur la plaque d'impact arrière (16) ;
dans lequel des impacts sur les plaques d'impact avant et arrière (15, 16) transmettent une force sur l'adaptateur de broche (1) dans une première et une seconde direction, la seconde direction étant sensiblement colinéaire et opposée à la première direction.

3. Outil d'impact orthopédique selon l'une quelconque revendication précédente, dans lequel l'ensemble moteur (8, 12) comprend un mécanisme bielle-manivelle.

4. Outil d'impact orthopédique selon l'une quelconque revendication précédente, dans lequel l'ensemble moteur (8, 12) comprend un moteur à bobine mobile.

5. Outil d'impact orthopédique selon l'une quelconque revendication précédente, dans lequel :
l'outil d'impact orthopédique comprend un capteur de position accouplé de manière fonctionnelle à l'une parmi l'unité de commande de l'outil d'impact orthopédique et l'ensemble moteur (8, 12) de l'outil d'impact orthopédique, dans lequel le capteur est configuré pour déterminer si l'outil est poussé vers un objet ou éloigné d'un objet, le capteur est configuré pour amener l'unité de commande ou l'ensemble moteur (8,12) de l'outil d'impact orthopédique à diriger l'élément d'impact (4) pour qu'il transmette une force plus importante sur la plaque d'impact avant (15) et une force moindre sur la plaque d'impact arrière (16) lorsqu'il est détecté que l'outil est poussé vers un objet, et le capteur est configuré pour amener l'unité de commande ou l'ensemble moteur (8, 12) de l'outil d'impact orthopédique à diriger l'élément d'impact (4) pour qu'il transmette une force plus importante sur la plaque d'impact avant (15) et une force moindre sur la plaque d'impact arrière (16) lorsqu'il est détecté que l'outil est poussé vers un objet.

6. Outil d'impact orthopédique selon l'une quelconque revendication précédente, dans lequel l'outil comprend un capteur de torsion configuré pour :
déterminer un mouvement latéral de l'outil d'impact orthopédique ; et
signaler un tel mouvement latéral.

7. Outil d'impact orthopédique selon l'une quelconque revendication précédente, dans lequel l'outil d'impact orthopédique comprend en outre un élément d'éclairage disposé sur une surface extérieure de l'outil d'impact orthopédique, l'élément d'éclairage étant couplé de manière opérationnelle à l'unité de commande.

8. Outil d'impact orthopédique selon l'une quelconque revendication précédente, dans lequel la vitesse de l'élément d'impact (4) peut être commandée par la vitesse à laquelle l'air est comprimé ou décompressé.

9. Outil d'impact orthopédique selon l'une quelconque revendication précédente, dans lequel l'adaptateur de broche (1) a un ajustement configuré pour décaler une broche, un ciseau, un alésoir ou un autre instrument chirurgical fixé de l'axe central de l'outil d'impact orthopédique tout en maintenant une orientation parallèle de la broche, du ciseau, de l'alésoir ou d'un autre instrument chirurgical par rapport à l'axe central de l'outil d'impact orthopédique.

10. Outil d'impact orthopédique selon la revendication 1, comprenant en outre un cran (10) pour maintenir l'élément d'impact (4) dans une position.

11. Outil d'impact orthopédique selon la revendication 10, dans lequel :
l'unité de commande est configurée pour ordonner à l'ensemble moteur (8, 12) de provoquer un mouvement du piston (6) et une compression d'air dans la chambre à air, de sorte que, lorsque la pression de l'air comprimé dépasse une force du cran (10), l'élément d'impact (4) sera amené à se déplacer d'une première position à une seconde position, frappant ainsi la plaque d'impact avant (15) ou arrière (16).

12. Outil d'impact orthopédique selon la revendication 10 ou 11, dans lequel une force de retenue dudit cran (10) peut être commandée par un solénoïde qui est accouplé de manière fonctionnelle à l'unité de commande.

13. Outil d'impact orthopédique selon l'une quelconque des revendications 10 à 12, comprenant en outre une commande d'ajustement de force accouplée de manière fonctionnelle à l'unité de commande, dans lequel la commande d'ajustement de force peut être utilisée pour au moins l'une parmi la modification de la vitesse à laquelle ledit piston (6) se déplace dans la chambre à air, et la modification de la force de rétention du cran (10).

14. Outil d'impact orthopédique selon l'une quelconque revendication précédente, dans lequel l'adaptateur de broche (1) est proche de la plaque d'impact avant (15) de l'élément d'enclume (14) et distal par rapport à la plaque d'impact arrière (16) de l'élément d'enclume (14).
